# EUROPEAN PATENT APPLICATION

(11) **EP 2 554 992 A1**
(43) Date of publication of application: **06.02.2013**
(21) Application number: 11817647.8
(22) Date of filing: 18.08.2011
(51) Int. Cl.: G01N 33/558

(54) **MULTIASSAY IMMUNOCHROMATOGRAPHIC CHIP**

(30) Priority: 19.08.2010 CN 201010257719
(71) Applicant: Institute of Microbiology and Epidemiology, Academy of Military Medical Sciences, PLA, Beijing 100071 (CN)
(72) Inventor: ZHOU, Lei, Beijing 100071 (CN); GUO, Zhaobiao, Beijing 100071 (CN); YANG, Ruifu, Beijing 100071 (CN)
(74) Representative: HOFFMANN EITLE
(86) International application number: PCT/CN2011/001376
(87) International publication number: WO 2012/022119

(57) **Abstract**

A multiassay immunochromatographic chip comprises: a viscous bottom lining (1), a sample pad (2), a bonding pad (3), an analysis membrane (4) and a water absorption pad (5).The bonding pad (3) fixedly has a plurality of assay binding substances (6) and a control binding substance (7), the assay binding substance (6) is formed by joining a tracer (8) and a liquid-phase detection probe (9), the control binding substance (7) is formed by joining the tracer (8) and a liquid-phase control probe (11); the analysis membrane (4) is provided with a detection matrix unit (12), each detection matrix unit comprises a detection zone (13) and a control zone (14), wherein the detection zone (13) consists of a plurality of solid phase detection probes (15) and the control zone (14) consists of a solid phase control probe (16). Organic integration of immunochromatographic reaction modes and chip assay matrix settings enables high throughput assay of multiple target substances with one sample load.

## Description

### Technical Field

The invention pertains to the technical field of immunologic diagnosis, and relates to a multiassay (or "multi-detecting") immunochromatographic chip, which integrates immunochromatography assay and chip assay and can achieve synchronous detection of multiple target substances with one sample load.

### Background of the Invention

Immunochromatography assay is a mature on-site rapid detecting technology, and an immunochromatographic strip comprises the following parts in physical structure: a laminating card (or "viscous bottom lining") [a], a sample pad [b], a conjugate pad (or "bonding pad") [c], an analysis membrane [d], and an water absorption pad [e], wherein, a kind of marker-biomolecule conjugate (or "binding substance") [f] is fixed to the conjugate pad, and one kind of biomolecule is fixed to the analysis membrane [d] as a test line [g] and aother kind of biomolecule is fixed as a control line [h]. The sample pad [b], conjugate pad [c], analysis membrane [d], and water absorption pad [e] are fixed to the laminating card [a] in a certain overlapping relation, and thereby the flow continuity of liquid in the immunochromatographic strip is ensured. During the detection, once the operator adds a liquid sample in droplets onto the sample pad [b], the sample infiltrates into the conjugate pad [c], so that the fixed conjugate [f] is re-dissolved and becomes free, and moves toward the water absorption pad [e] through the test line [g] and control line [h] under the driving effect of the water absorption pad [e] and capillarity action. In that process, specific immunologic reactions occur on the test line [g] and control line [h] and thereby detectable signals [j] are generated, depending on the existence of target substances in the sample. Owing to the fact that all biological reagents (f, g, h) required for the detection have been fixed in the strip, the operator only has to perform the addition of liquid sample, and the entire testing process takes only 10-15 minutes. Therefore, the immunochromatography assay is the easiest and rapidest on-site detecting technology. However, it usually can only be used to analyze one target substance with one sample load, and cannot achieve a high throughput detection.

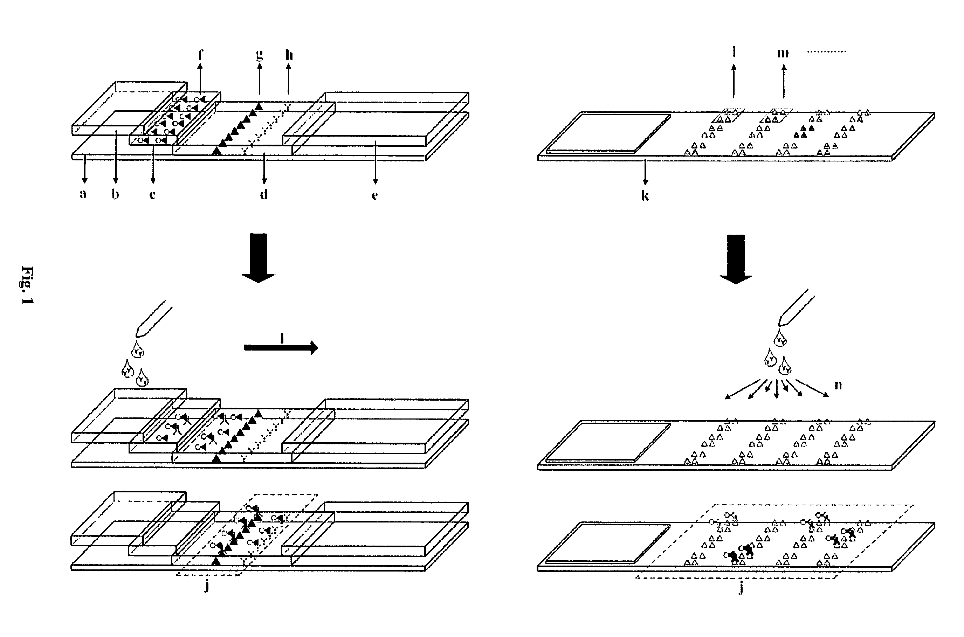

Chip assay has emerged in response to the demand for high throughput detection of target substances (nucleic acids or proteins) in biological analysis, wherein, nucleic acids or proteins that are used as detection probes are fixed to different areas ([1] [m]......) of a glass substrate [k] by a micro-arrayer so as to form detecting array (or "matrix"), and each area on the array is used for detection of one kind of target substance. The sample is directly added in droplets on the detecting array of the chip, and after a series of treatments including incubation (to accomplish nucleic acid hybridization or immunologic reaction), rinsing, and tracing, etc., so that detectable signals [j] can be generated in a specific area on the array of the chip, depending on the existence of a certain kind of target substance. Different from the homogeneous mode of the immunochromatography assay where only one sample loading cycle is required, the chip assay, especially protein chip assay, employs a heterogeneous mode, and thus inevitably requires repetitive incubation and rinsing steps in the process of operation; further, the chip assay has stringent requirements for standardization and environment of operation. Therefore, the chip assay cannot meet the demand for on-site detection or even on-site laboratory. The distinguishing characteristics between immunochromatographic assay and chip assay are shown in Figure 1.

If a new technology that incorporates the rapidness and convenience of immunochromatography assay and the high throughput of chip assay seamlessly and can achieve high throughput on-site detection through simple and convenient operations is developed, the demand of on-site inspection, detecting and supervision departments (e.g., disease control system) for high throughput and rapid screening can be met better.

### Summary of the Invention

The object of the invention is to disclose a multiassay immunochromatographic chip, which can overcome the disadvantages in the prior art that the immunochromatography assay cannot accomplish high throughput detection and the chip assay cannot be used on-site due to high operation complexity, and integrates the reaction mode of immunochromatography assay and the array setting of chip assay seamlessly, and thereby achieves high throughput on-site detection through easy operation, i.e., synchronous detection of multiple target substances with one sample load.

The object of the invention is attained with the following technical solutions.

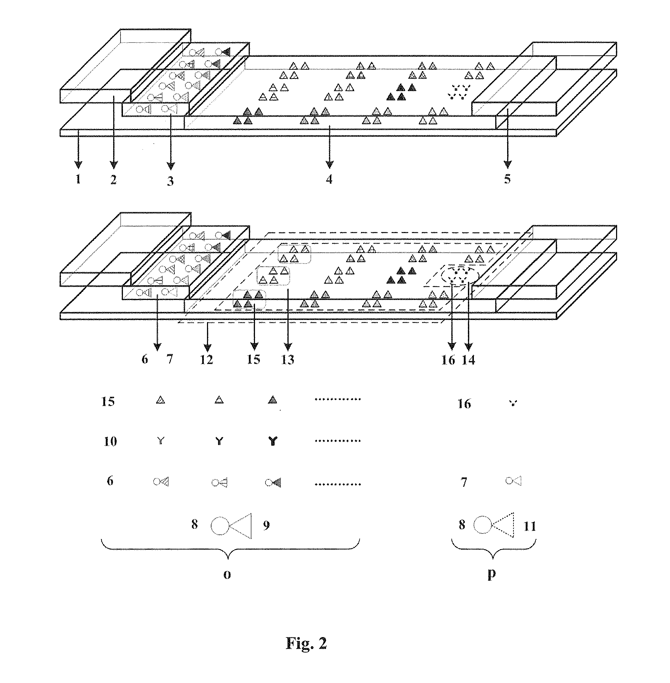

In structure, the immunochromatographic chip in the invention comprises a laminating card (or "viscous bottom lining") [1], a sample pad [2], a conjugate pad (or "bonding pad") [3], an analysis membrane [4], and a water absorption pad [5] (as shown in Figure 2).

Wherein, the laminating card [1] is an object made of a rigid material and coated with a pressure sensitive adhesive on one surface, and the object is PVC plate.

Wherein, the sample pad [2] is an object that has a large bed volume and uniform microstructure, and the object is selected from water absorption paper, cellulose membrane, glass fiber, non-woven fabric, or blood filtering membrane.

Wherein, the conjugate pad [3] is an object that has a large bed volume and a uniform microstructure, and the object is selected from glass fiber, polyester membrane, or non-woven fabric; several kinds of test conjugates (or "assay binding substances") [6] and a control conjugate (or "control binding substance") [7] are fixed to the conjugate pad [3]; each kind of the test conjugates [6] is formed by conjugating a marker (or "joining a tracer") [8] with one kind of liquid-phase test probe (or "liquid-phase detection probe") [9], and is in specific one-to-one correspondence to a certain kind of detected target (or "target to be detected") [10]; the control conjugate [7] is formed by conjugating the marker [8] with a liquid-phase control probe [11], and can control whether the immunochromatographic analysis is in a normal state.

Wherein, the analysis membrane [4] is an object that has a uniform microstructure, and the object is selected from nitrocellulose membrane or nylon membrane; the analysis membrane [4] is provided with a test array unit (or "detection matrix unit") [12]; the test array unit [12] comprises a test zone (or "detection zone") [13] and a control spot (or "control zone") [14]; the test zone [13] comprises various kinds of solid-phase test probes (or "solid phase detection probe") [15], and the control spot [14] comprises a solid-phase control probe [16]; each kind of the solid-phase test probes [15] on the test zone [13] is positioned fixedly at a well-defined position and corresponds to the specific detection of a certain kind of detected target [10], and the solid-phase control probe [16] on the control spot [14] is positioned fixedly at a well-defined position to control whether the entire immunochromatographic analysis process is in a normal state.

Wherein, the water absorption pad [5] is an object that has a large bed volume, and the object is selected from water absorption paper or cellulose membrane.

Wherein, each detected target [10] corresponds to two test probes. One of the test probes is fixed as a solid-phase test probe [15] on the analysis membrane [4], and the other test probe as a liquid-phase test probe [9] is conjugated with the marker [8] to form a test conjugate [6] fixed on the conjugate pad [3].

Wherein, each kind of the solid-phase test probes [15] is positioned fixedly at well-defined position on the test zone [13] of the analysis membrane [4], and corresponds to specific detection of a certain kind of detected target [10] individually. Each kind of the solid-phase test probes [15] may be specifically immunologically reacted with a certain kind of detected target [10] and the corresponding kind of liquid-phase test probe [9] of the test conjugate [6], so that the amount of the marker [8] bonded at the well-defined position where this kind of solid-phase test probe [15] is located is changed by the immunologic reaction, and thereby the existence and concentration of this kind of detected target [10] can be revealed by the amount of the marker [8].

Wherein, the solid-phase control probe [16] is positioned fixedly at a well-defined position on the control spot [14] of the analysis membrane [4], and can bond directly with the liquid-phase control probe [11] of the control conjugate [7] to control whether the immunochromatographic analysis process is in a normal state.

Wherein, the immunochromatographic chip in the invention comprises a sandwich immunochromatographic chip, an indirect immunochromatographic chip, and a competitive immunochromatographic chip; wherein, the sandwich immunochromatographic chip comprises a double-antibody sandwich immunochromatographic chip for antigen detection and a double-antigen sandwich immunochromatographic chip for antibody detection; the indirect immunochromatographic chip is used for detection of specific antibodies in blood serum sample, with the test conjugate formed by conjugating the marker with the secondary antibody of the detected antibodies; the competitive immunochromatographic chip is used for the detection of small-molecule substances, such as a hapten that has only one antigenic determinant.

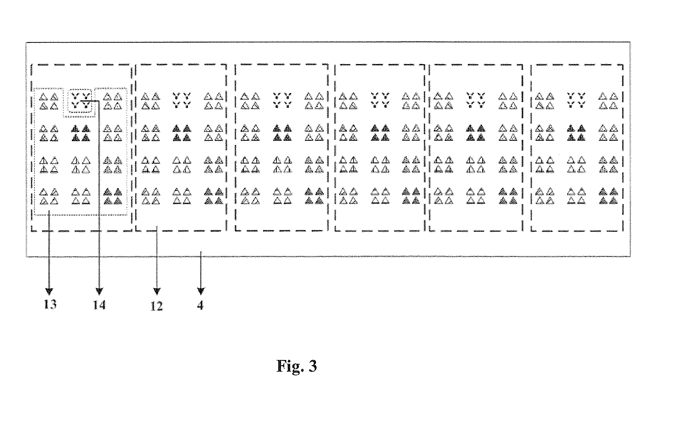

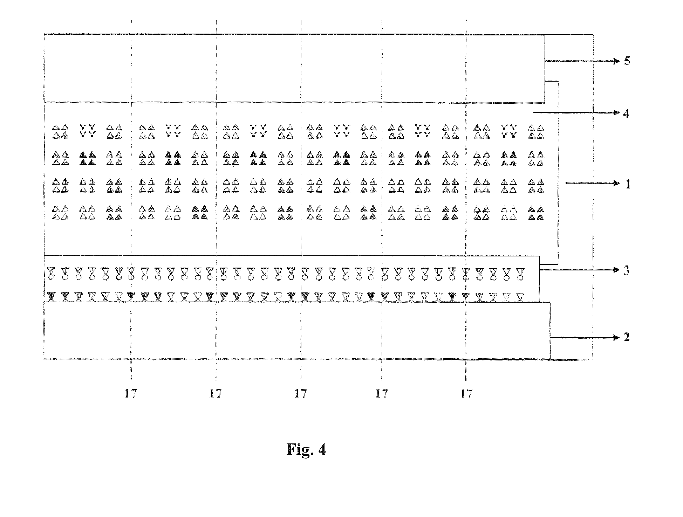

The method for preparation of the immunochromatographic chip in the invention comprises the following steps:
A. Preparation of a conjugate pad [3]: mixing a control conjugate [7] with various kinds of test conjugates [6] to obtain a mixture of conjugates, applying the mixture on a piece of fiber glass, polyester membrane, or non-woven fabric that is used as the conjugate pad [3], and drying the resultant for future use;
B. Preparation of an analysis membrane [4]: adding respectively various kinds of antigens that serve as solid-phase test probes [15] and an antibody that serves as solid-phase control probe [16] in the form of round spots on a nitrocellulose membrane or a nylon membrane at well-defined, fixed and addressable positions, to form a test zone [13] and a control spot [14] respectively, and thereby form a test array unit [12]; and drying for future use. Wherein, in the test array unit [12], the various kinds of solid-phase test probes [15] and their well-defined fixed positions are in one-to-one correspondence to certain kinds of detected targets [10], and only one solid-phase control probe [16] is required, which also has a well-defined fixed position. In a better manner, a plurality of test array units [12] are prepared on the analysis membrane [4] (see Figure 3).
C. Laminating and cutting: laminating the sample pad [2], conjugate pad [3], analysis membrane [4], and water absorption pad [5] in sequence to a PVC plate serving as the laminating card [1], while ensuring overlapping relation between each parts, so as to obtain the finished immunochromatographic chip in the invention; the finished immunochromatographic chip can be used directly or loaded into a plastic cartridge for use. In a better manner, the PVC plate is cut into separate and usable finished products at the break points [17] between the test array units [12] (see Figure 4).

A method for biological target detection with the immunochromatographic chip in the invention, comprising:
A. Adding sample: adding a liquid sample or pretreated liquid sample in droplets onto the sample pad [2] of the immunochromatographic chip in the invention;
B. Immunochromatographic reaction: standing for several minutes, till the immunochromatographic reaction is completed. In the immunochromatographic reaction process, specific immunologic reactions occur among the test conjugates [6], detected targets [10], and solid-phase test probes [15], and the bonded amount of the marker [8] is changed at the well-defined fixed positions on the analysis membrane [4]. The bonded amount of the marker [8] at a certain position directly reflects the existence or concentration of a certain kind of detected target [10];
C. Result judgment: judging the result directly by visual examination for a color marker [8], or judging the result with instruments for a marker [8] that generates an optical, electrical, or magnetic signal. Since one kind of solid-phase test probe [15] for a certain kind of detected target [10] is positioned fixedly at a well-defined position on the analysis membrane [4], this kind of detected target [10] can be detected qualitatively and quantitatively by judging the signals including a color, optical, electrical, or magnetic signal from the marker [8] fixed at the well-defined position.

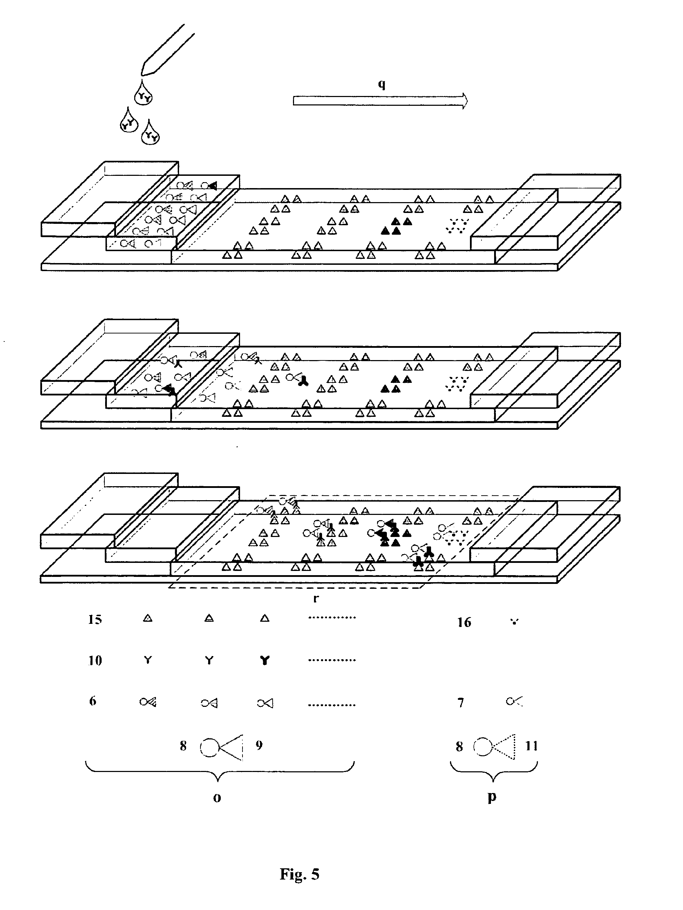

The detection principle of the immunochromatographic chip in the invention is as follows: as shown in Figure 5, in the detecting process, after the liquid sample is added to the sample pad [2], the liquid sample infiltrates through the sample pad [2] into the conjugate pad [3]; under the action of the base material of the liquid sample, the test conjugates [6] and the control conjugate [7] fixed in the conjugate pad [3] are re-dissolved and become free, and leave the conjugate pad [3] together with all kinds of detected targets [10] in the sample and enter into the analysis membrane [4]; under the action of capillarity, these substances flow through the test zone [13] and control spot [14] toward the water absorption pad [5]; in that process, each kind of detected targets [10] and a corresponding kind of test conjugates [6] are specifically immunologically reacted with a corresponding kind of solid-phase test probes [15] fixed at the well-defined position on the test zone [13], depending on the mode of immunologic reaction, and the control conjugate [7] bonds directly with the solid-phase control probe [16] on the control spot [14]; therefore, through the specific immunologic reaction among the solid-phase test probe [15] fixed at a well-defined position on the test zone [13] of the analysis membrane [4], the detected target [10], and the liquid-phase test probe [9] of the test conjugates [6], the amount of the marker [8] at the well-defined fixed position on the test zone [13] of the analysis membrane [4] is changed; ultimately, the existence and intensity of the signal from the marker [8] at the well-defined fixed position refers to the existence and concentration of the detected target [10]; through the direct bonding between the solid-phase control probe [16] fixed at a well-defined position on the control spot [14] of the analysis membrane [4] and the liquid-phase control probe [11] of the control conjugate [7], the marker [8] is bonded at the well-defined fixed position on the control spot [14] of the analysis membrane; the existence of the marker [8] indicates that the immunochromatographic analysis is in a normal state.

In the conventional art, the immunochromatography assay cannot achieve a high throughput detection, and the chip assay cannot be used on-site due to complex operation. To solve those problems, in the invention, an immunochromatographic chip is designed by integrating the reaction mode of immunochromatography assay and the array setting of chip assay seamlessly, ultimately achieving a high throughput detection which is easy to use on site, i.e., synchronous detection of multiple target substances (i.e., various kinds of detected targets) with one sample load.

### Brief description of the figures:

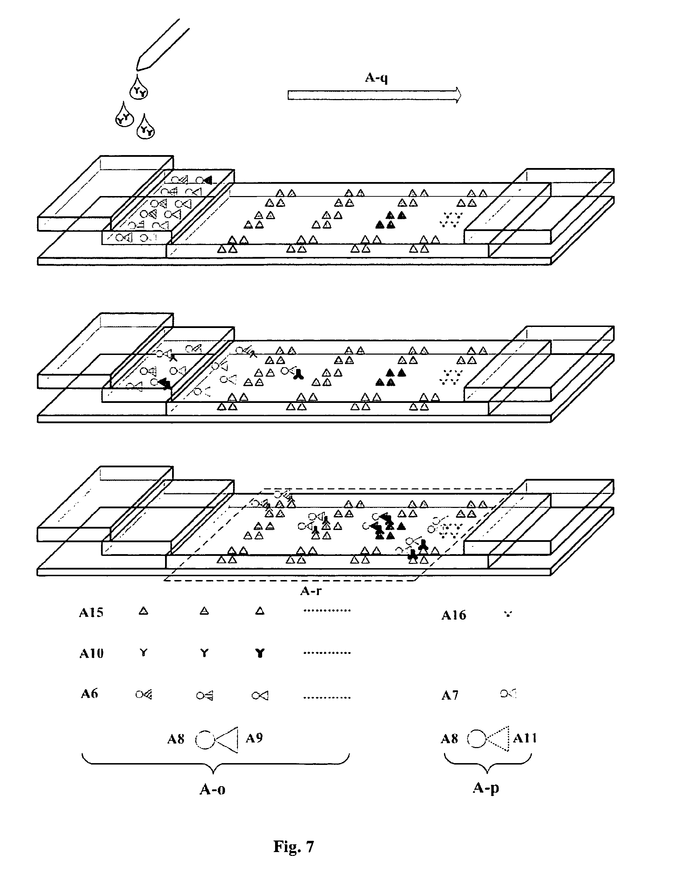

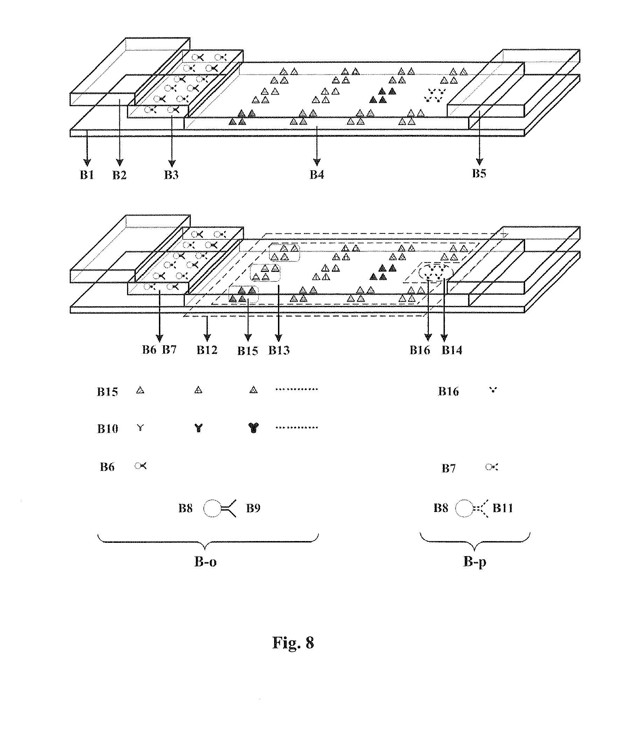

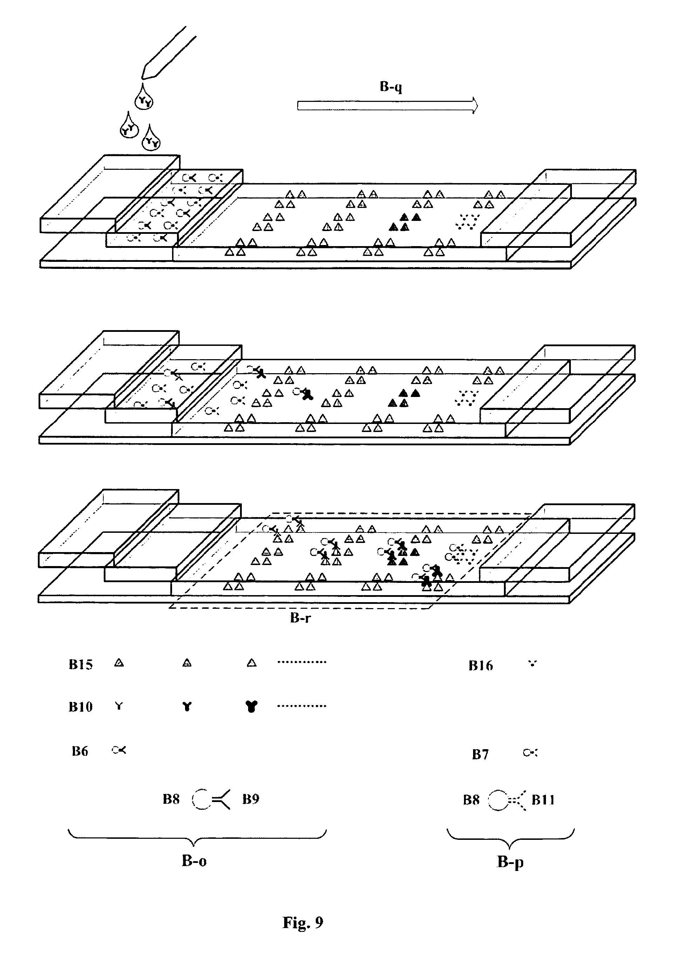

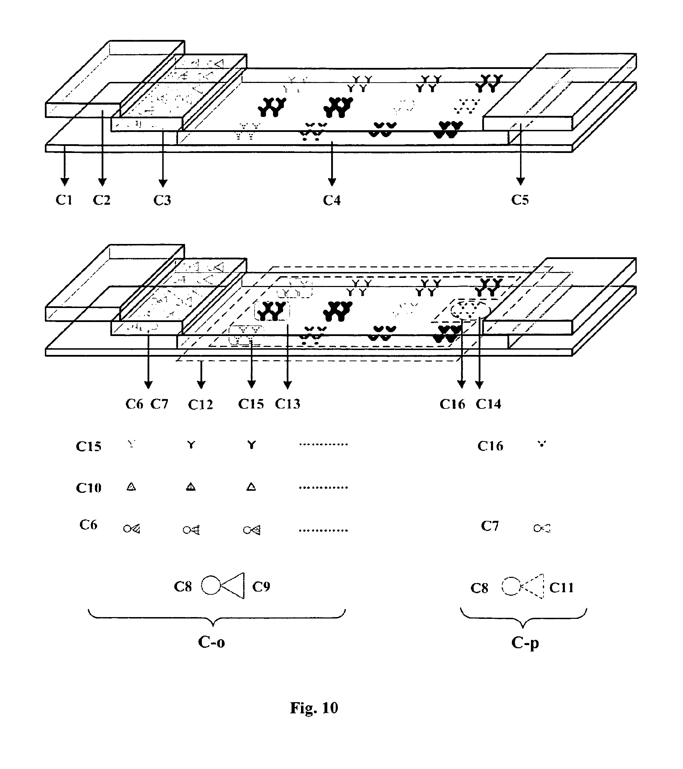

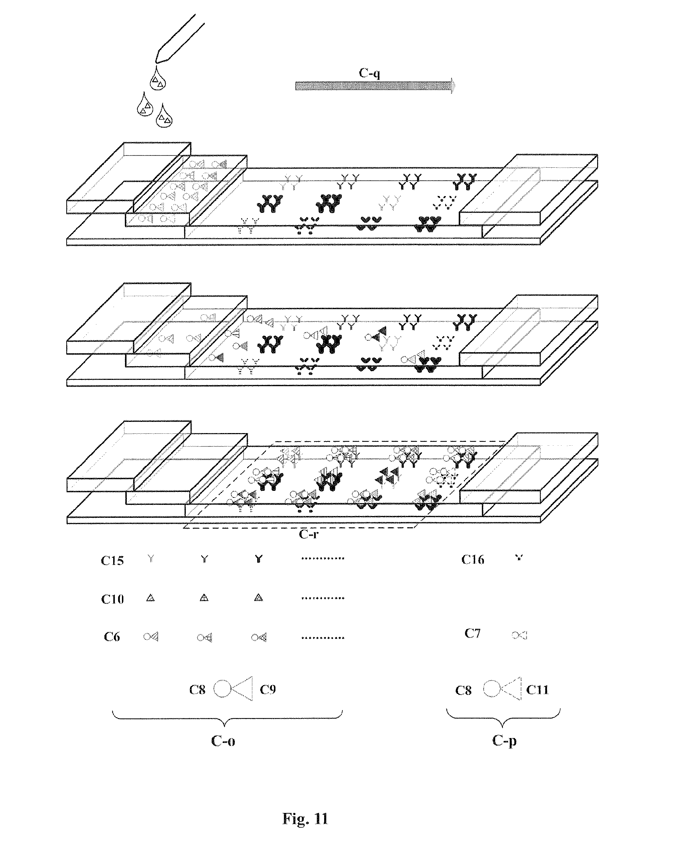

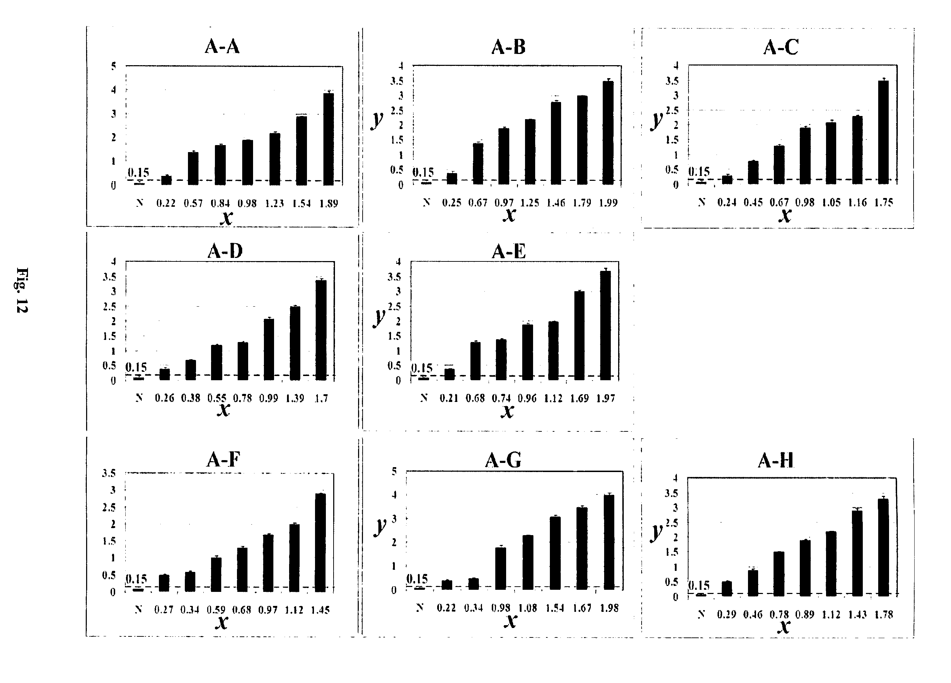

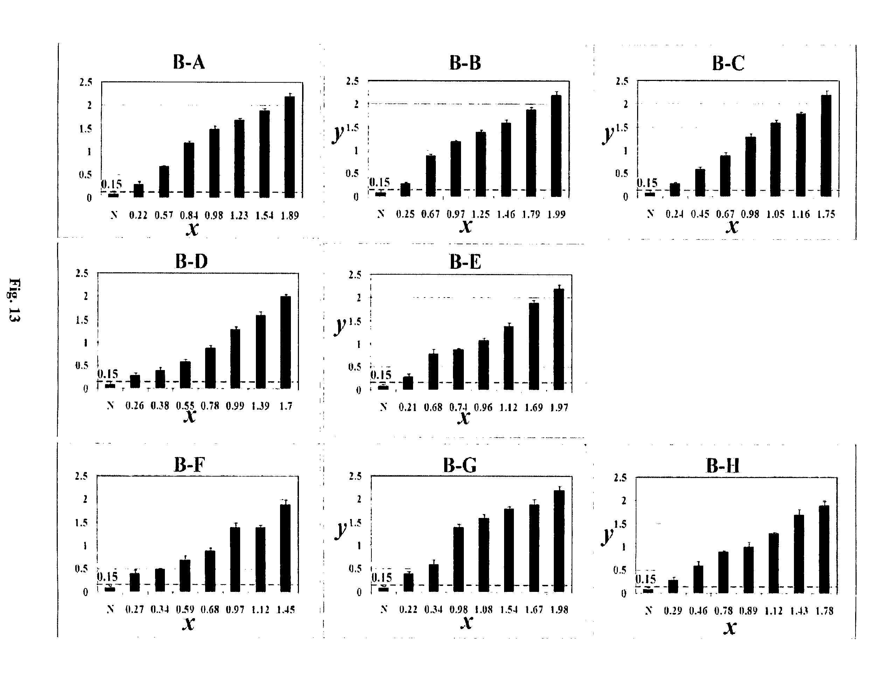

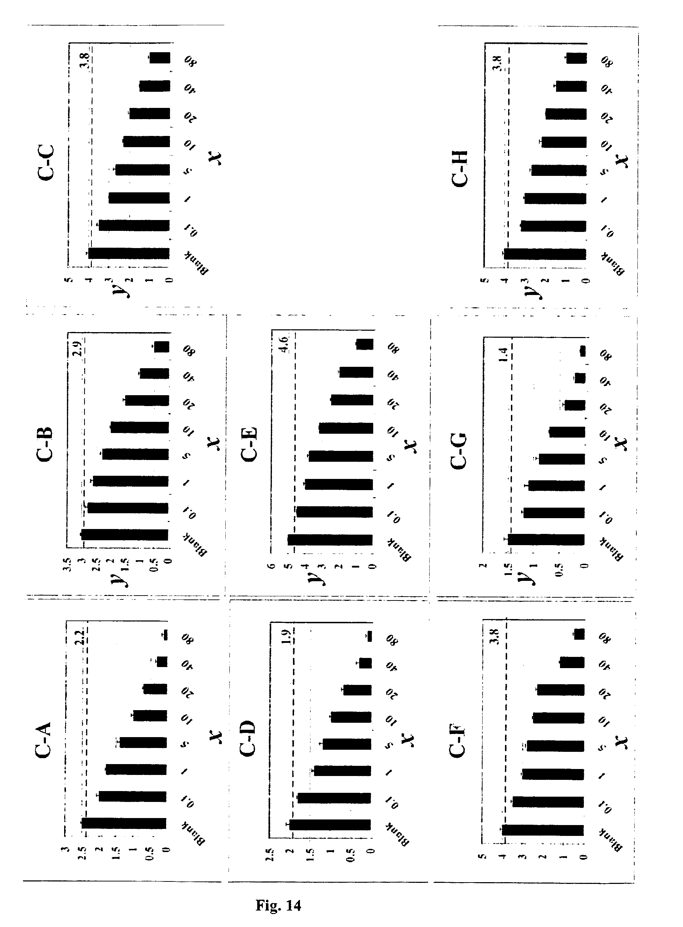

Figure 1 illustrates comparison between immunochromatography assay and chip assay;
   a. Laminating card, b. Sample pad, c. Conjugate pad, d. Analysis membrane, e. Water absorption pad, f. Conjugate, g. Test line, h. Control line, i. Flow direction of liquid sample, j. Detectable signal, k. Glass substrate, 1. Test spot for target 1, m. Test spot for target 2, n. Diffusion direction of liquid sample
Figure 2 illustrates a structural sketch of the immunochromatographic chip;
   1. Laminating card, 2. Sample pad, 3. Conjugate pad, 4. Analysis membrane, 5. Water absorption pad, 6. Test conjugate, 7. Control conjugate, 8. marker, 9. Liquid-phase test probe, 10. detected target, 11. Liquid-phase control probe, 12. Test array unit, 13. Test zone, 14. Control spot, 15. Solid-phase test probe, 16. Solid-phase control probe
   O. Specific detection, p. System control
Figure 3.is a schematic diagram of preparation of the analysis membrane;
   4. Analysis membrane, 12. Test array unit, 13. Test zone, 14. Control spot
Figure 4 is a schematic diagram of laminating and cutting of the immunochromatographic chip;
   1. Laminating card, 2. Sample pad, 3. Conjugate pad, 4. Analysis membrane, 5. Water absorption pad, 17. Break point
Figure 5 is a diagram illustrating detection principle of the immunochromatographic chip;
   6. Test conjugate, 7. Control conjugate, 8. Marker, 9. Liquid-phase test probe, 10. Detected target, 11. Liquid-phase control probe, 15. Solid-phase test probe, 16.
      Solid-phase control probe
   O. Specific detection, p. System control, q. Flow direction of liquid sample, r. Detectable signal
Figure 6 illustrates a structural sketch of the double-antigen sandwich immunochromatographic chip ;
   A1. Laminating card, A2. Sample pad, A3. Conjugate pad, A4. Analysis membrane, A5. Water absorption pad, A6. Test conjugate, A7. Control conjugate, A8. Marker, A9. Liquid-phase test antigen, A10. Detected antibody, A11. Liquid-phase control antigen, A12. Test array unit, A13. Test zone, A14. Control spot, A15. Solid-phase test antigen, A16. Solid-phase control antibody
   A-o. Specific detection, A-p. System control
Figure 7 is a diagram illustrating detection principle of the double-antigen sandwich immunochromatographic chip;
   A6. Test conjugate, A7. Control conjugate, A8. Marker, A9. Liquid-phase test antigen, A10. Detected antibody, A11. Liquid-phase control antigen, A15. Solid-phase test antigen, A16. Solid-phase control antibody
   A-o. Specific detection, A-p. System control, A-q. Flow direction of liquid sample, A-r. Detectable signal
Figure 8 illustrates a structural sketch of the indirect immunochromatographic chip;
   B1. Laminating card, B2. Sample pad, B3. Conjugate pad, B4. Analysis membrane, B5. Water absorption pad, B6. Test conjugate, B7. Control conjugate, B8. Marker, B9. Goat Anti-Human IgG, B10. Detected Human antibody, B11. Goat Anti-Rabbit IgG, B12. Test array unit, B13. Test zone, B14. Control spot, B15. Solid-phase test antigen, B16. Solid-phase control antibody
   B-o. Specific detection, B-p. System control
Figure 9 is a diagram of detection principle of the indirect immunochromatographic chip;
   B6. Test conjugate, B7. Control conjugate, B8. Marker, B9. Goat Anti-Human IgG, B10. Detected human antibody, B11. Goat Anti-Rabbit IgG, B15. Solid-phase test antigen, B16. Solid-phase control antibody
   B-o. Specific detection, B-p. System control, B-q. Flow direction of liquid sample, B-r. Detectable signal
Figure 10 illustrates a structural sketch of the competitive immunochromatographic chip;
   C1. Laminating card, C2. Sample pad, C3. Conjugate pad, C4. Analysis membrane, C5. Water absorption pad, C6. Test conjugate, C7. Control conjugate, C8. Marker, C9. Liquid-phase test antigen, C10. Detected antigen, C11. Digoxin, C12. Test array unit, C13. Test zone, C14. Control spot, C15. Solid-phase test antibody, C16. Solid-phase control antibody
   C-o. Specific detection, C-p. System control
Figure 11 is a diagram illustrating detection principle of the competitive immunochromatographic chip;
   C6. Test conjugate, C7. Control conjugate, C8. Marker, C9. Liquid-phase test antigen, C10. Detected antigen, C11. Digoxin, C15. Solid-phase test antibody, C16. Solid-phase control antibody
   C-o. Specific detection, C-p. System control, C-q. Flow direction of liquid sample, C-r. Detectable signal
Figure 12 illustrates a result of an experimental example of the double-antigen sandwich immunochromatographic chip;
   A-A. Detection of antibody against influenza virus, A-B. Detection of antibody against Parainfluenza virus, A-C. Detection of antibody against Respiratory syncytial virus, A-D. Detection of antibody against *Mycoplasma pneumoniae,* A-E. Detection of antibody against *Chlamydia pneumoniae,* A-F. Detection of antibody against *Legionella pneumophilia,* A-G. Detection of antibody against *Hemophilus influenza,* A-H. Detection of antibody against *Klebsiella pneumoniae, x.* OD measurement by ELISA, *y*. T/C measurement by immunochromatographic chip
Figure 13 illustrates a result of an experimental example of the indirect immunochromatographic chip;
   B-A. Detection of antibody against Influenza virus, B-B. Detection of antibody against Parainfluenza virus, B-C. Detection of antibody against Respiratory syncytial virus, B-D. Detection of antibody against *Mycoplasma pneumoniae,* B-E. Detection of antibody against *Chlamydia pneumoniae,* B-F. Detection of antibody against *Legionella pneumophilia,* B-G. Detection of antibody against *Hemophilus influenza,* B-H. Detection of antibody against *Klebsiella pneumoniae, x.* OD measurement by ELISA, *y*. T/C measurement by immunochromatographic chip
Figure 14 illustrates a result of an experimental example of the competitive immunochromatographic chip
   C-A. Detection of Opium, C-B. Detection of Morphine, C-C. Detection of Heroin, C-D. Detection of Cocaine, C-E. Detection of Coca leaf, C-F. Detection of Amphetamine, C-G. Detection of Metamfetamine, C-H. Detection of Mezcaline, x. Concentration (ng/ml), *y*. T/C measurement by immunochromatographic chip

The invention will be further described in detaile by the following experimental examples and embodiments, but the invention is not limited thereto.

### Experimental example 1: Double-antigen sandwich immunochromatographic chip for the detection of "antibodies against suspectable pathogens related with fever of unknown origin"

Antibodies against influenza virus, parainfluenza virus, respiratory syncytial virus, *Mycoplasma pneumoniae, Chlamydia pneumoniae, Legionella pneumophilia, Hemophilus influenza,* and *Klebsiella pneumoniaepneumoniae* in the blood were detected with the double-antigen sandwich immunochromatographic chip, to rapidly determine the cause for fever of unknown origin for patients suffered therefrom.

### Method of preparation:

A. Preparation of a conjugate pad [A3]: conjugating influenza virus surface antigen A, parainfluenza virus surface antigen A, respiratory syncytial virus surface antigen A, *Mycoplasma pneumoniae* surface antigen A, *Chlamydia pneumoniae* surface antigen A, *Legionella pneumophilia* surface antigen A, *Hemophilus influenza* surface antigen A, and *Klebsiella pneumoniae* surface antigen A with fluorescein Cy5 respectively, to prepare 8 kinds of test conjugates [A6]; conjugating digoxin with Cy5, to prepare a control conjugate [A7]; mixing the control conjugate [A7] with the test conjugates [A6] in PB buffer solution (0.03 M, pH=7.2) to form a mixture of conjugates with the concentration of each of them at 1 mg/ml; adding the mixture of conjugates in droplets on a piece of 1 cm×30 cm glass fiber used as the conjugate pad [A3], and drying the resultant for 5 h at 37□ for future use;
B. Preparation of an analysis membrane [A4]: taking influenza virus surface antigen B, parainfluenza virus surface antigen B, respiratory syncytial virus surface antigen B, *Mycoplasma pneumoniae* surface antigen B, *Chlamydia pneumoniae* surface antigen B, *Legionella pneumophilia* surface antigen B, *Hemophilus influenza* surface antigen B, and *Klebsiella pneumoniae* surface antigen B as the solid-phase test antigens [A15]; taking rabbit anti-digoxin IgG as the solid-phase control antibody [A16]; adding above-mentioned 9 biomolecules (or "bioactive molecules") at a concentration of 0.1 mg/ml in the form of round spots with a dispensing rate of 15 µl/spot, on a piece of nitrocellulose membrane with a size of 2.5 cm×30 cm to form a 2.5 cm×3 cm test array unit [A12], wherein, the 8 kinds of solid-phase test antigens [A15] constituted a test zone [A13], and the one solid-phase control antibody [A16] constituted a control spot [A14]; preparing 10 test array units [A12] consecutively, and drying them for future use;
C. Laminating and cutting: laminating a 1.5 cm×30 cm sample pad [A2], a 1 cm×30 cm conjugate pad [A3], a 2.5 cm×30 cm analysis membrane [A4], and a 3 cm×30 cm water absorption pad [A5] to a 7.4 cm×30 cm PVC plate that serves as the laminating card [A1] in sequence, wherein, the sample pad [A2] was laminated above the conjugate pad [A3] with an overlap of 3 mm, the conjugate pad [A3] was laminated above the analysis membrane [A4] with an overlap of 1 mm, and the water absorption pad [A5] was laminated above the analysis membrane [A4] with an overlap of 2 mm; finally obtaining a 7.4 cm×30 cm semi-finished product, wherein, the points at 3 cm interval in longitudinal direction were taken as the break points [17] between the test array units [A12]; cutting the semi-finished product in the invention at the break points [17] to obtain separate and usable finished products, so as to obtain 10 immunochromatographic chips in the invention;

### The sample detecting process and results:

A. Adding sample: mixing 100 µl blood serum sample with 900 µl sample diluent buffer (0.03 M PB with pH=7.2, containing 0.5% PEG20000, 0.05% SDS, and 2% BSA), and then adding 1,000 µl mixture in droplets onto the sample pad [A2] of the immunochromatographic chip;
B. Immunochromatographic reaction: standing for 5min, till the immunochromatographic reaction was completed;
C. Judgment of result: scanning the fluorescent signal of Cy5 on the immunochromatographic chip with a scanner; obtaining a signal intensity corresponding to each detected antibody [A10] by collecting and analyzing the signal from the marker [A8] at the well-defined fixed position on the test zone [A13] since the position of solid-phase test antigen [A15] for each detected antibody [A10] was fixed and well-defined on the analysis membrane [A4], and assigning the signal intensity as T; assigning the signal intensity on the control spot [A14] as C; taking T/C as the testing result of each detected antibody [A10];
D. Assessment of detection performance: assessing the detection performance of the immunochromatographic chip with clinical positive or negative antibody samples against influenza virus, parainfluenza virus, respiratory syncytial virus, *Mycoplasma pneumoniae, Chlamydia pneumoniae, Legionella pneumophilia, Hemophilus influenza,* and *Klebsiella pneumoniae,* and comparing the result of immunochromatographic chip with that of ELISA (wherein, samples with OD<0.2 in ELISA were negative ones, while samples with OD>0.2 were positive ones). The result (shown in Figure 12) demonstrated that, with T/C=0.15 as the cut-off value, the immunochromatographic chip can distinguish positive and negative clinical samples accurately, and the sensitivity was comparable with that of ELISA.

### Experimental example 2: Indirect immunochromatographic chip for the detection of "antibodies against suspectable pathogens related with fever of unknown origin"

Antibodies against influenza virus, parainfluenza virus, respiratory syncytial virus, *Mycoplasma pneumoniae, Chlamydia pneumoniae, Legionella pneumophilia, Hemophilus influenza,* and *Klebsiella pneumoniae* in the blood were detected with the indirect immunochromatographic chip, to determine the cause for fever of unknown origin for patients suffered therefrom.

### Method of preparation:

A. Preparation of a conjugate pad [B3]: conjugating Goat Anti-Human IgG with fluorescein Cy5 to prepare a test conjugate [B6]; conjugating Goat Anti-Rabbit IgG with Cy5 to prepare a control conjugate [B7]; mixing the control conjugate [B7] and the test conjugate [B6] in PB buffer solution (0.03 M, pH=7.2) to form a mixture of conjugates, wherein, the final concentration of the test conjugate [B6] was 8 mg/ml, and the final concentration of the control conjugate [B7] was 1 mg/ml; adding the mixture of conjugates in droplets onto a piece of 1 cm×30 cm glass fiber used as the conjugate pad [B3], and drying the resultant for 5 h at 37□ for future use;
B. Preparation of an analysis membrane [A4]: taking influenza virus surface antigen B, parainfluenza virus surface antigen B, respiratory syncytial virus surface antigen B, *Mycoplasma pneumoniae* surface antigen B, *Chlamydia pneumoniae* surface antigen B, *Legionella pneumophilia* surface antigen B, *Hemophilus influenza* surface antigen B, and *Klebsiella pneumoniae* surface antigen B as the solid-phase test antigens [B15]; taking rabbit IgG as the solid-phase control antibody [B16]; adding the 9 biomolecules at a concentration of 0.1 mg/ml in the form of round spots with a dispensing rate of 15 µl/spot on a piece of nitrocellulose membrane with a size of 2.5 cm×30 cm to form a 2.5 cm×3 cm test array unit [B12], wherein, the 8 kinds of solid-phase test antigens [A15] constituted a test zone [B13], and the one solid-phase control antibody [B16] constituted a control spot [B14]; preparing 10 test array units [B12] consecutively, and drying them for future use;
C. Laminating and cutting: laminating a 1.5 cm×30 cm sample pad [B2], a 1 cm×30 cm conjugate pad [B3], a 2.5 cm×30 cm analysis membrane [B4], and a 3 cm×30 cm water absorption pad [B5] to a 7.4 cm×30 cm PVC plate that serves as the laminating card [B1] in sequence, wherein, the sample pad [B2] was laminated above the conjugate pad [B3] with a overlap of 3 mm, the conjugate pad [B3] was laminated above the analysis membrane [B4] with a overlap of 1 mm, and the water absorption pad [B5] was laminated above the analysis membrane [B4] with a overlap of 2 mm; finally obtaining a 7.4 cm×30 cm semi-finished product, wherein, the points at 3 cm interval in longitudinal direction were taken as break points [17] between the test array units [B12]; cutting the semi-finished product at the break points [17] to obtain separate and usable finished products, so as to obtain the immunochromatographic chips in the invention;

### The sample detecting process and results:

A. Adding sample: mixing 100 µl blood serum sample with 900 µl sample diluent buffer (0.03 M PB with pH=7.2, containing 0.5% PEG20000, 0.05% SDS, and 2% BSA), and then adding 1000 µl mixture in droplets onto the sample pad [B2] of immunochromatographic chip;
B. Immunochromatographic reaction: standing for 5min., till the immunochromatographic reaction was completed;
C. Judgment of result: scanning the fluorescent signal of Cy5 on the immunochromatographic chip with a scanner; obtaining a signal intensity corresponding to each detected antibody [B 10] by collecting and analyzing the signal from the marker [B8] at the well-defined fixed position on the test zone [B13] since the position of solid-phase test antigen [B15] for each detected antibody [B10] was fixed and well-defined on the analysis membrane [B4], and assigning the signal intensity as T; assigning the signal intensity on the control spot [B14] as C; taking T/C as the testing result of each detected antibody [B10];
D. Assessment of detection performance: assessing the detection performance of the immunochromatographic chip with clinical positive or negative antibody samples against influenza virus, parainfluenza virus, respiratory syncytial virus, mycoplasma pneumoniae, *Chlamydia pneumoniae, Legionella pneumophilia, Hemophilus influenza,* and *Klebsiella pneumoniae,* and comparing the result of immunochromatographic chip with that of ELISA (wherein, samples with OD<0.2 in ELISA were negative ones, while samples with OD>0.2 were positive ones). The result (shown in Figure 13) demonstrated that: with T/C=0.15 as the cut-off value, the immunochromatographic chip can distinguish positive and negative clinical samples accurately, and the sensitivity was comparable with that of ELISA.

### Experimental example 3: Competitive immunochromatographic chip for the detection of "illegal drugs"

The trace of illegal drugs including opium, morphine, heroin, cocaine, coca leaf, amphetamine, metamfetamine, and mezcaline in urine was detected with the competitive immunochromatographic chip.

### Method of preparation:

A. Preparation of a conjugate pad [C3]: conjugating BSA-opium, BSA-morphine, BSA-heroin, BSA-cocaine, BSA-coca leaf, BSA-amphetamine, BSA-metamfetamine, and BSA-mezcaline with fluorescein Cy5 respectively, to prepare 8 kinds of test conjugates [C6]; conjugating digoxin with Cy5, to prepare a control conjugate [C7]; mixing the control conjugate [C7] with the test conjugates [C6] in PB buffer solution (0.03 M, pH=7.2) to form a mixture of conjugates with the concentration of each of them at 1 mg/ml; adding the mixture of conjugates in droplets onto a piece of 1 cm×30 cm glass fiber used as the conjugate pad [C3], and drying the resultant for 5 h at 37□ for future use;
B. Preparation of an analysis membrane [C4]: taking anti-opium monoclonal antibody, anti-morphine monoclonal antibody, anti-heroin monoclonal antibody, anti-cocaine monoclonal antibody, anti-coca leaf monoclonal antibody, anti-amphetamine monoclonal antibody, anti-metamfetamine monoclonal antibody, and anti-mezcaline monoclonal antibody as the solid-phase test antibodies [C15]; taking rabbit anti-digoxin IgG as the solid-phase control antibody [C16]; adding the 9 biomolecules at a concentration of 0.1 mg/ml in the form of round spots with a dispense rate of 15 µl/spot on a piece of 2.5 cm×30 cm nitrocellulose membrane to form a 2.5 cm×3 cm test array unit [C12], wherein, the 8 kinds of solid-phase test antibodies [C15] constituted a test zone [C13], and the one solid-phase control antibody [C16] constituted a control spot [C14]; preparing 10 test array units [C12] consecutively, and drying them for future use;
C. Laminating and cutting: laminating a 1.5 cm×30 cm sample pad [C2], a 1 cm×30 cm conjugate pad [C3], a 2.5 cm×30 cm analysis membrane [C4], and a 3 cm×30 cm water absorption pad [C5] to a 7.4 cm×30 cm PVC plate that serves as the laminating card [C1] in sequence, wherein, the sample pad [C2] was laminated above the conjugate pad [C3] with an overlap of 3 mm, the conjugate pad [C3] was laminated above the analysis membrane [C4] with an overlap of 1 mm, and the water absorption pad [C5] was laminated above the analysis membrane [C4] with an overlap of 2 mm; finally obtaining a 7.4 cm×30 cm semi-finished product, wherein, the points at 3 cm interval in longitudinal direction were taken as break points [17] between the test array units [C12]; cutting the semi-finished product at the break points [17] to obtain separate and usable finished products, so as to obtain the immunochromatographic chips in the invention;

### The sample detecting process and results:

A. Adding sample: mixing 100 µl urine sample with 900 µl sample diluent buffer (0.03 M PB with pH=7.2, containing 0.5% PEG8000, 0.1% SDS, and 1% BSA), and then adding 1,000 µl mixture in droplets onto the sample pad [C2] of immunochromatographic chip;
B. Immunochromatographic reaction: standing for 5min., till the immunochromatographic reaction was completed;
C. Judgment of result: scanning the fluorescent signal of Cy5 on the immunochromatographic chip with a scanner; obtaining a signal intensity corresponding to each detected antigen [C10] by collecting and analyzing the signal from the marker [C8] at the well-defined fixed position on the test zone [C13] since the position of solid-phase test antibody [C15] for each detected antigen [C10] was fixed and well-defined on the analysis membrane [C4], and assigning the signal intensity as T; assigning the signal intensity on the control spot [C14] as C; taking T/C as the testing result of detected antigen [C10];
D. Assessment of detection performance: diluting serially standard samples of opium, morphine, heroin, cocaine, coca leaf, amphetamine, metamfetamine, and mezcaline with urine that is negative to illegal drugs, and assessing the detection performance of the immunochromatographic chip; the result (as shown in Figure 14) demonstrated that: the immunochromatographic chip can distinguish positive and negative samples accurately, and the sensitivity reached 100 pg/ml.

### Detailed Description of the Embodiments

### Embodiment 1: Double-antigen sandwich immunochromatographic chip

The double-antigen sandwich immunochromatographic chip was used to detect specific antibodies in blood serum samples, wherein, the test conjugate was formed by conjugating a marker with the specific antigen for the detected antibody.

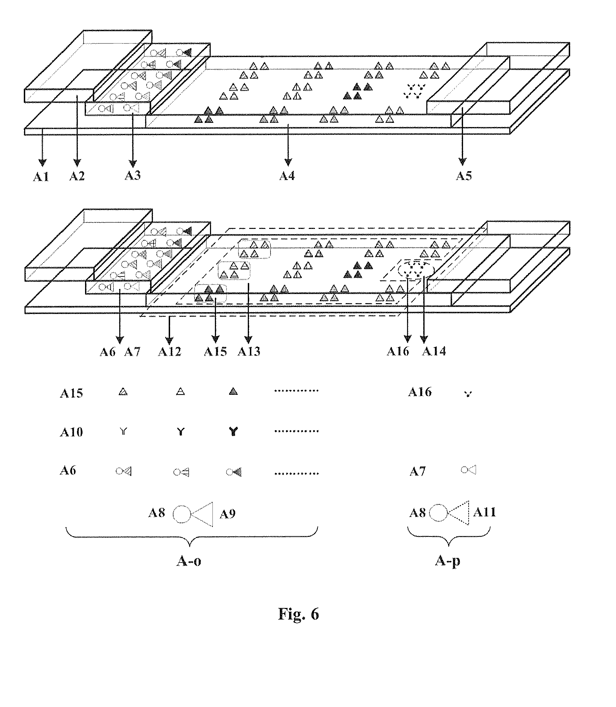

### Structure of the double-antigen sandwich immunochromatographic chip (shown in Figure 6)

The double-antigen sandwich immunochromatographic chip comprises a laminating card [A1], a sample pad [A2], a conjugate pad [A3], an analysis membrane [A4], and an water absorption pad [A5];
the laminating card [A1] is an object made of rigid material and coated with a pressure sensitive adhesive on one surface, such as a PVC plate, on which the sample pad [A2], conjugate pad [A3], analysis membrane [A4], and water absorption pad [A5] can be laminated and fixed in an appropriate overlapping relation, so as to ensure the flow continuity of the liquid in the double-antigen sandwich immunochromatographic chip;
the sample pad [A2] is a piece of water absorption paper, where the liquid sample is added when the double-antigen sandwich immunochromatographic chip is used;
the conjugate pad [A3] is a piece of glass fiber, to which several kinds of test conjugates [A6] and a control conjugate [A7] are fixed; wherein, each kind of the test conjugate [A6] is formed by conjugating a marker [A8] with one kind of liquid-phase test antigen [A9], and in specific one-to-one correspondence to a certain kind of detected antibody [A10]; the control conjugate [A7] is formed by conjugating the marker [A8] with a liquid-phase control antigen [A11], and can control whether the immunochromatographic analysis is in a normal state;

The analysis membrane [A4] is a piece of nitrocellulose membrane, wherein, the analysis membrane [A4] is provided with a test array unit [A12], and the test array unit [A12] comprises a test zone [A13] and a control spot [A14]; the test zone [A13] comprises various kinds of solid-phase test antigens [A15], and the control spot [A14] comprises a solid-phase control antibody [A16]; each kind of solid-phase test antigens [A15] on the test zone [A13] is positioned fixedly at a well-defined position, and corresponds to the specific detection of a certain kind of detected antibody [A10], and the solid-phase control antibody [A16] on the control spot [A14] is positioned fixedly at a well-defined position to control whether the entire immunochromatographic analysis is in a normal state;

The water absorption pad [A5] is a piece of water absorption paper.

### The method for preparation of the double-antigen sandwich immunochromatographic chip was as follows:

A. Preparation of a conjugate pad [A3]: mixing a control conjugate [A7] with various kinds of test conjugates [A6] to obtain a mixture of conjugates, applying the mixture of conjugates on a piece of glass fiber used as the conjugate pad [A3], and drying the resultant for future use;
B. Preparation of an analysis membrane [A4]: adding respectively various kinds of solid-phase test antigens [A15] and a solid-phase control antibody [A16] in the form of round spots on a nitrocellulose membrane at well-defined, fixed, and addressable positions, to form a test zone [A13] and a control spot [A14] respectively, and thereby form a test array unit [A12]; preparing a plurality of test array units [A12] consecutively on the analysis membrane [A4] and drying them for future use. Wherein, in the test array unit [A12], the various kinds of solid-phase test antigens [A15] and their well-defined fixed positions were in one-to-one correspondence to certain kinds of the detected antibodies [A10], and only one solid-phase control antibody [A16] was required which also had a well-defined fixed position.
C. Laminating and cutting: laminating the sample pad [A2], conjugate pad [A3], analysis membrane [A4], and water absorption pad [A5] in sequence to a PVC plate serving as the laminating card [A1], while ensuring overlapping relation between each parts; cutting the PVC plate into separate and usable finished products at the break points [17] between the test array units [A12], so as to obtain finished immunochromatographic chip in the invention; the finished immunochromatographic chip can be used directly or loaded into a plastic cartridge for use.

### The method for biological target detection with the double-antigen sandwich immunochromatographic chip in the invention was as follows.

A. Adding sample: adding a liquid sample or a pretreated liquid sample in droplets onto the sample pad [A2] of the immunochromatographic chip in the invention;
B. Immunochromatographic reaction: standing for several minutes, till the immunochromatographic reaction was completed; in the immunochromatographic reaction process, specific immunologic reactions occurred among the test conjugates [A6], the detected antibodies [A10], and the solid-phase test antigens [A15], and the bonded amount of the marker [A8] was changed at the well-defined fixed positions on the analysis membrane [A4]; the bonded amount of the marker [A8] at a certain position directly reflected the existence or concentration of a certain kind of detected antibody [A10];
C. Result judgment: judging the result directly by visual examination for a color marker [A8] or judging the result with instruments for a marker [A8] that generated an optical, electrical, or magnetic signal. Since one kind of solid-phase test antigen [A15] for a certain kind of detected antibody [A10] was positioned fixedly at a well-defined position on the analysis membrane [A4], this kind of detected antibody [A10] can be detected qualitatively and quantitatively by judging the signal including a color, optical, electrical, or magnetic signal from the marker [A8] fixed at the well-defined position.

### Detection principle of the double-antigen sandwich immunochromatographic chip in the invention (shown in Figure 7)

In the detecting process, after the liquid sample was added to the sample pad [A2], the liquid sample infiltrated through the sample pad [A2] into the conjugate pad [A3]; under the action of the base material of liquid sample, the test conjugates [A6] and control conjugate [A7] fixed in the conjugate pad [A3] were re-dissolved and became free, and left the conjugate pad [A3] together with all kinds of detected antibodies [A10] in the sample and entered into the analysis membrane [A4]; under the action of capillarity, these substances flowed through the test zone [A13] and control spot [A14] toward the water absorption pad [A5]; in that process, each kind of test conjugates [A6] bonded specifically with a site of a certain kind of detected antibody [A10], while at the same time another site of this kind of detected antibody [A10] bonded specifically with the corresponding kind of solid-phase test antigen [A15] fixed at a well-defined position on the test zone [A13], and the control conjugate [A7] bonded directly with the solid-phase control antibody [A16] on the control spot [A14]; therefore, through the specific double-antigen sandwich immunologic reaction among the solid-phase test antigen [A15] fixed at a well-defined position on the test zone [A13] of the analysis membrane [A4], the detected antibody [A10], and the liquid-phase test antigen [A9] of test conjugate [A6], the amount of the marker [A8] at the well-defined fixed position on the test zone[A13] of the analysis membrane [A4] was changed; ultimately, the existence and intensity of the signal from the marker [A8] at the well-defined fixed position referred to the existence and concentration of the detected antibody [A10], wherein, the signal intensity of the marker [A8] was proportional to the concentration of the detected antibody [A10]; through the direct bonding between the solid-phase control antibody [A16] fixed at a well-defined position on the control spot [A14] of the analysis membrane [A4] and the liquid-phase control antigen [A11] of the control conjugate [A7], the marker [A8] was bonded at the well-defined fixed position on the control spot [A14] of the analysis membrane [A4]; the existence of the marker [A8] indicates that the immunochromatographic analysis was in a normal state.

### Embodiment 2: Indirect immunochromatographic chip

The indirect immunochromatographic chip was used to detect specific antibodies in blood serum samples, wherein, the test conjugate was formed by conjugating a marker with the secondary antibody of the detected antibodies. Hereafter the embodiment will be described using an example of detection of human blood serum samples.

### Structure of the indirect immunochromatographic chip (shown in Figure 8)

The indirect immunochromatographic chip comprises a laminating card [B1], a sample pad [B2], a conjugate pad [B3], an analysis membrane [B4], and an water absorption pad [B5];
the laminating card [B1] is an object made of rigid material and coated with a pressure sensitive adhesive on one surface, such as a PVC plate, on which the sample pad [B2], conjugate pad [B3], analysis membrane [B4], and water absorption pad [B5] can be laminated and fixed in an appropriate overlapping relation, so as to ensure the flow continuity of the liquid in the indirect immunochromatographic chip;
the sample pad [A2] is a piece of cellulose membrane, where the liquid sample is added when the indirect immunochromatographic chip is used;
the conjugate pad [B3] is a piece of polyester membrane, to which a test conjugate [B6] and a control conjugate [B7] are fixed; wherein, the test conjugate [B6] is formed by conjugating a marker [B8] with Goat Anti-Human IgG [B9], and may be specifically reacted with the detected human antibody [B10]; the control conjugate [B7] is formed by conjugating the marker [B8] with Goat Anti-Rabbit IgG [B11], and can control whether the immunochromatographic analysis is in a normal state;
the analysis membrane [B4] is a piece of nylon membrane, wherein, the analysis membrane [B4] is provided with a test array unit [B12], and the test array unit [B12] comprises a test zone [B 13] and a control spot [B 14]; the test zone [B 13] comprises various kinds of solid-phase test antigens [B 15], and the control spot [B 14] comprises a solid-phase control antibody [B16], i.e., Rabbit Anti-IgG; each kind of solid-phase test antigens [B15] on the test zone [B13] is positioned fixedly at a well-defined position and corresponds to the specific detection of a certain kind of detected human antibody [B10], and the solid-phase control antibody [B16] on the control spot [B14] is positioned fixedly at a well-defined position to control whether the entire immunochromatographic analysis is in a normal state;

The water absorption pad [B5] is a piece of cellulose membrane.

### The method for preparation of the indirect immunochromatographic chip was as follows.

A. Preparation of a conjugate pad [B3]: mixing a control conjugate [B7] with a test conjugate [B6] to obtain a mixture of conjugates, applying the mixture of conjugates on a piece of polyester membrane that was used as the conjugate pad [B3], and drying the resultant for future use;
B. Preparation of an analysis membrane [B4]: adding respectively various kinds of solid-phase test antigens [B 15] and a solid-phase control antibody [B16] in the form of round spots on a nylon membrane at well-defined, fixed, and addressable positions, to form a test zone [B13] and a control spot [B14] respectively, and thereby form a test array unit [B12]; preparing a plurality of test array units [B12] consecutively on the analysis membrane [B4], and drying them for future use. Wherein, in the test array unit [B12], the various kinds of solid-phase test antigens [B15] and their well-defined fixed positions were in one-to-one correspondence to certain kinds of detected human antibodies [B10], and only one solid-phase control antibody [B16] was required which also had a well-defined position;
C. Laminating and cutting: laminating the sample pad [B2], conjugate pad [B3], analysis membrane [B4], and water absorption pad [B5] in sequence to a PVC plate serving as the laminating card [B1], while ensuring overlapping relation between each parts; cutting the PVC plate into separate and usable finished products at the break points [17] between the test array units [B12], so as to obtain finished immunochromatographic chip in the invention; the finished immunochromatographic chip can be used directly or loaded into a plastic cartridge for use.

### The method for biological target detection with the indirect immunochromatographic chip in the invention was as follows.

A. Adding sample: adding a liquid sample or a pretreated liquid sample in droplets onto the sample pad [B2] of the immunochromatographic chip in the invention;
B. Immunochromatographic reaction: standing for several minutes, till the immunochromatographic reaction was completed; in the immunochromatographic reaction process, specific immunologic reactions occurred among the test conjugate [B6], the detected human antibodies [B10], and the solid-phase test antigens [B15], and the bonded amount of the marker [B8] was changed at the well-defined fixed positions on the analysis membrane [B4]; the bonded amount of the marker [B8] at a certain position directly reflected the existence or concentration of a certain kind of detected human antibody [B10];
C. Result judgment: judging the result directly by visual examination for a color marker [B8] or judging the result with instruments for a marker [B8] that generated an optical, electrical, or magnetic signal. Since one kind of solid-phase test antigen [B 15] for a certain kind of detected human antibody [B10] was positioned fixedly at a well-defined position on the analysis membrane [B4], this kind of detected human antibody [B10] can be detected qualitatively and quantitatively by judging the signals including a color, optical, electrical, or magnetic signal from the marker [B8] fixed at the well-defined position.

### Detection principle of the indirect immunochromatographic chip in the invention (shown in Figure 9)

In the detecting process, after the liquid sample was added to the sample pad [B2], the liquid sample infiltrated through the sample pad [B2] into the conjugate pad [B3]; under the action of the base material of the liquid sample, the test conjugate [B6] and control conjugate [B7] fixed in the conjugate pad [B3] were re-dissolved and became free, and left the conjugate pad [B3] together with all kinds of detected human antibodies [B10] in the sample and entered into the analysis membrane [B4]; under the action of capillarity, these substances flowed through the test zone [B13] and control spot [B14] toward the water absorption pad [B5]; in that process, the test conjugate [B6] bonded specifically with a site of a specific kind of detected human antibodies [B10], while at the same time another site of this kind of detected human antibodies [B10] bonded specifically with a corresponding kind of solid-phase test antigens [B15] fixed at the well-defined position on the test zone [B13], and the control conjugate [B7] bonded directly with the solid-phase control antibody [B16], i.e., Rabbit Anti-IgG, on the control spot [B14]; therefore, through the specific immunologic reaction among the solid-phase test antigen [B15] fixed at a well-defined position on the test zone [B13] of the analysis membrane [B4], the detected human antibody [B10], and the Goat Anti-Human IgG [B9] of test conjugate [B6], the amount of the marker [B8] at the well-defined fixed position on the test zone [B13] of the analysis membrane [B4] was changed; ultimately, the existence and intensity of the signal from the marker [B8] at the well-defined fixed position referred to the existence and concentration of the detected human antibody [B10], wherein, the signal intensity of the marker [B8] was proportional to the concentration of the detected human antibody [B10]; through the direct bonding between the solid-phase control antibody [B16] (i.e., Rabbit Anti-IgG) fixed at a well-defined position on the control spot [B14] of the analysis membrane [B4] and the Goat Anti-Rabbit IgG [B11] of control conjugate [B7], the marker [A8] was bonded at the well-defined fixed position on the control spot [B14] of the analysis membrane [B4]; the existence of the marker [B8] indicated that the immunochromatographic analysis was in a normal state.

### Embodiment 3: Competitive immunochromatographic chip

The competitive immunochromatographic chip was used to detect the small-molecules, such as a hapten that has only one antigenic determinant.

### Structure of the competitive immunochromatographic chip (shown in Figure 10)

The competitive immunochromatographic chip comprises a laminating card [C1], a sample pad [C2], a conjugate pad [C3], an analysis membrane [C4], and an water absorption pad [C5];
the laminating card [C1] is an object made of rigid material and coated with a pressure sensitive adhesive on one surface, such as a PVC plate, on which the sample pad [C2], conjugate pad [C3], analysis membrane [C4], and water absorption pad [C5] can be laminated and fixed in an appropriate overlapping relation, so as to ensure the flow continuity of the liquid in the competitive immunochromatographic chip;
the sample pad [C2] is a piece of glass fiber, where the liquid sample is added when the competitive immunochromatographic chip is used;
the conjugate pad [C3] is a piece of non-woven fabric, to which several kind of test conjugates [C6] and a control conjugate [C7] are fixed; wherein, each kind of the test conjugates [C6] is formed by conjugating a marker [C8] with one kind of liquid-phase test antigen [C9], and is in specific one-to-one correspondence to a certain kind of detected antigen [C10] and has an antigenic determinant same as this kind of detected antigen [C10]; the control conjugate [C7] is formed by conjugating the marker [C8] with digoxin [C11], and can control whether the immunochromatographic analysis is in a normal state;
the analysis membrane [C4] is a piece of nitrocellulose membrane, wherein, the analysis membrane [C4] is provided with a test array unit [C12], and the test array unit [C12] comprises a test zone [C13] and a control spot [C14]; the test zone [C13] comprises various kinds of solid-phase test antibodies [C15], and the control spot [C14] comprises a solid-phase control antibody [C16]; each kind of solid-phase test antibodies [C15] on the test zone [C13] is positioned fixedly at a well-defined position, and corresponds to the specific detection of a certain kind of detected target [C10], and the solid-phase control antibody [A16] (i,e., Rabbit Anti-digoxin) on the control spot [C14] is positioned fixedly at a well-defined position to control whether the entire immunochromatographic analysis is in a normal state;

The water absorption pad [C5] is a piece of water absorption paper.

### The method for preparation of the competitive immunochromatographic chip was as follows:

A. Preparation of a conjugate pad [C3]: mixing a control conjugate [C7] with various kinds of test conjugates [C6] to obtain a mixture of conjugates, applying the mixture of conjugates on a piece of non-woven fabric that was used as the conjugate pad [C3], and drying the resultant for future use;
B. Preparation of an analysis membrane [C4]: adding respectively various kinds of solid-phase test antibodies [C15] and a solid-phase control antibody [C16] in the form of round spots on a nylon membrane at well-defined, fixed, and addressable positions, to form a test zone [C13] and a control spot [C14] respectively, and thereby form a test array unit [C12]; preparing a plurality of test array units [C12] consecutively on the analysis membrane [C4], and drying them for future use. Wherein, in the test array unit [C12], the various kinds of solid-phase test antibodies [C15] and their well-defined fixed positions were in one-to-one correspondence to certain kinds of detected antigens [C10], and only one solid-phase control antibody [C16] was required which also had a well-defined position;
C. Laminating and cutting: laminating the sample pad [C2], conjugate pad [C3], analysis membrane [C4], and water absorption pad [C5] in sequence to a PVC plate serving as laminating card [C1], while ensuring overlapping relation between each parts, cutting the PVC plate in the invention into separate and usable finished products at the break points [17] between the test array units [C12],so as to obtain finished immunochromatographic chip in the invention; the finished immunochromatographic chip can be used directly or loaded into a plastic cartridge for use.

### The method for biological target detection with the competitive immunochromatographic chip in the invention was as follows:

A. Adding sample: adding a liquid sample or a pretreated liquid sample in droplets onto the sample pad [C2] of the immunochromatographic chip in the invention;
B. Immunochromatographic reaction: standing for several minutes, till the immunochromatographic reaction was completed; in the immunochromatographic reaction process, specific immunologic reactions occurred among the test conjugates [C6], the detected antigens [C10] and the solid-phase test antibodies [C15], and the bonded amount of the marker [C8] was changed at the well-defined fixed positions on the analysis membrane [C4]; the bonded amount of the marker [C8] at a each position directly reflected the existence or concentration of a certain kind of detected antigen [C10];
C. Result judgment: judging the result directly by visual examination for a color marker [A8] or judging the result with instruments for a marker [A8] that generated an optical, electrical, or magnetic signal. Since one kind of solid-phase test antibody [C15] for a certain kind of detected antigen [C10] was positioned fixedly at a well-defined position on the analysis membrane [C4], this kind of detected antigen [C10] can be detected qualitatively and quantitatively by judging the signals including a color, optical, electrical, or magnetic signal from the marker [C8] fixed at the well-defined position.

### Detection principle of the competitive immunochromatographic chip in the invention (shown in Figure 11)

In the detecting process, after the liquid sample was added to the sample pad [C2], the liquid sample infiltrated through the sample pad [C2] into the conjugate pad [C3]; under the action of the base material of the liquid sample, the test conjugates [C6] and the control conjugate [C7] fixed in the conjugate pad [C3] were re-dissolved and became free, and left the conjugate pad [C3] together with the all kinds of detected antigens [C10] in the sample and entered into the analysis membrane [C4]; under the action of capillarity, these substances flowed through the test zone [C13] and control spot [C14] toward the water absorption pad [C5]; in that process, each kind of test conjugates [C6] and a certain kind of detected antigen [C10] competed to bond specifically with the corresponding kind of solid-phase test antibody [C15] fixed at the well-defined position on the test zone [C13], and the control conjugate [C7] directly bonded with the solid-phase control antibody [C16] (i.e., Rabbit Anti-Digoxin) on the control spot [C14]; therefore, through the specific competitive immunologic reaction among the detected antigen [C10], the liquid-phase test antigen [C9] of test conjugate [C6], and the solid-phase test antibody [C15] fixed at a well-defined position on the test zone [C13] of the analysis membrane [C4], the amount of the marker [B8] at the well-defined fixed positions on the test zone [B13] of the analysis membrane [B4] was changed, i.e., each kind of the test conjugates [C6] occupied all binding sites of a corresponding kind of solid-phase detection antibodies [C15] and thereby resulted in the strongest signal of marker [C8] if a certain kind of detected antigens [C10] did not exist; whereas a certain kind of detected antigens [C10] competed with a corresponding kind of test conjugates [C6] for the binding site of a corresponding kind of solid-phase test antibodies [C15] and thereby resulted in weaker signal of marker [C8] if this kind of detected antigens [C10] existed; ultimately, the existence and intensity of the signal from the marker [C8] at the well-defined fixed position referred to the existence and concentration of a certain kind of detected antigen [C10], wherein, the signal intensity of the marker [C8] was inversely proportional to the concentration of this kind of detected antigen [C10]; through the direct bonding between the solid-phase control antibody [C16] (i.e., Rabbit Anti-Digoxin) fixed at a well-defined position on the control spot [C14] of the analysis membrane [C4] and the digoxin [C11] of control conjugate [C7], the marker [C8] was bonded at the well-defined fixed position on the control spot [C14] of the analysis membrane [C4]; the existence of the marker [C8] indicated that the immunochromatographic analysis was in a normal state.

## Claims

1. A multiassay immunochromatographic chip, comprising:
a laminating card [1], a sample pad [2], a conjugate pad [3], an analysis membrane [4], and an water absorption pad [5].

2. The multiassay immunochromatographic chip according to claim 1, wherein, the laminating card [1] is an object made of a rigid material and coated with a pressure sensitive adhesive on one surface, and the object is PVC plate.

3. The multiassay immunochromatographic chip according to claim 1, wherein, the sample pad [2] is an object that has a large bed volume and a uniform microstructure, and the object is selected from water absorption paper, cellulose membrane, fiber glass, non-woven fabric, or blood filtering membrane.

4. The multiassay immunochromatographic chip according to claim 1, wherein, the conjugate pad [3] is an object that has a large bed volume and a uniform microstructure, and the object is selected from glass fiber, polyester membrane, or non-woven fabric; several kinds of test conjugates [6] and a control conjugate [7] are fixed to the conjugate pad [3]; each kind of the test conjugates [6] is formed by conjugating a marker [8] with one kind of liquid-phase test probe [9], and is in specific one-to-one correspondence to a certain kind of detected target [10]; the control conjugate [7] is formed by conjugating the marker [8] with a liquid-phase control probe [11], and can control whether the immunochromatographic analysis is in a normal state.

5. The multiassay immunochromatographic chip according to claim 1, wherein, the analysis membrane [4] is an object that has a uniform microstructure, and the object is selected from nitrocellulose membrane or nylon membrane; the analysis membrane [4] is provided with a test array unit [12]; the test array unit [12] comprises a test zone [13] and a control spot [14]; the test zone [13] comprises various kinds of solid-phase test probes [15], and the control spot [14] comprises a solid-phase control probe [16]; each kind of the solid-phase test probes [15] on the test zone [13] is positioned fixedly at a well-defined position and corresponds to the specific detection of a certain kind of detected target [10], and the solid-phase control probe [16] on the control spot [14] is positioned fixedly at a well-defined position to control whether the entire immunochromatographic analysis is in a normal state.

6. The multiassay immunochromatographic chip according to claim 1, wherein, the water absorption pad [5] is an object that has a large bed volume, and the object is selected from water absorption paper or cellulose membrane.

7. The multiassay immunochromatographic chip according to claim 4 or 5, wherein, each detected target [10] corresponds to two test probes, wherein, one of the test probes is fixed as a solid-phase test probe [15] on the analysis membrane [4], and the other test probe as a liquid-phase test probe [9] is conjugated with a marker [8] to form a test conjugate [6] fixed on the conjugate pad [3].

8. The multiassay immunochromatographic chip according to claim 5, wherein, each kind of the solid-phase test probes [15] is positioned fixedly at well-defined position on the test zone [13] of the analysis membrane [4], and corresponds to specific detection of a certain kind of detected target [10] individually; each kind of the solid-phase test probes [15] is specifically immunologically reacted with a certain kind of detected target [10] and the corresponding kind of liquid-phase test probe [9] of the test conjugate [6], so that the amount of the marker [8] bonded at the well-defined position where this kind of solid-phase test probe [15] is located is changed by the immunologic reaction, and thereby the existence and concentration of this kind of detected target [10] is revealed by the amount of the marker [8].

9. The multiassay immunochromatographic chip according to claim 5, wherein, the solid-phase control probe [16] is positioned fixedly at a well-defined position on the control spot [14] of the analysis membrane [4], and bonds directly with the liquid-phase control probe [11] of the control conjugate [7] to control whether the immunochromatographic analysis is in a normal state.

10. The multiassay immunochromatographic chip according to claim 1, comprising a sandwich immunochromatographic chip, an indirect immunochromatographic chip, and a competitive immunochromatographic chip; wherein, the sandwich immunochromatographic chip comprises a double-antibody sandwich immunochromatographic chip for antigen detection and a double-antigen sandwich immunochromatographic chip for antibody detection; the indirect immunochromatographic chip is used for detection of specific antibodies in blood serum sample, with the test conjugate formed by conjugating a marker with a secondary antibody of detected antibodies; the competitive immunochromatographic chip is used for the detection of small-molecule substances, such as a hapten that has only one antigenic determinant.

11. A method for preparation of the multiassay immunochromatographic chip according to any one of claims 1-10, comprising the following steps:
A. Preparation of a conjugate pad [3]: mixing a control conjugate [7] with various kinds of test conjugates [6] to obtain a mixture of conjugates, applying the mixture on a piece of fiber glass, polyester membrane, or non-woven fabric that is used as the conjugate pad [3], and drying the resultant for future use;
B. Preparation of an analysis membrane [4]: adding respectively various kinds of antigens that serve as the solid-phase test probes [15] and an antibody that serves as the solid-phase control probe [16] in the form of round spots on a nitrocellulose membrane or a nylon membrane at well-defined, fixed and addressable positions, to form a test zone [13] and a control spot [14] respectively, and thereby form a test array unit [12]; and drying it for future use, wherein, in the test array unit [12], the various kinds of solid-phase test probes [15] and their well-defined fixed positions are in one-to-one correspondence to certain kinds of detected targets [10], and only one solid-phase control probe [16] is required, which also has a well-defined fixed position, and in a better manner, a plurality of test array units [12] are prepared on the analysis membrane [4].
C. Laminating and cutting: laminating the sample pad [2], conjugate pad [3], analysis membrane [4], and water absorption pad [5] in sequence to a PVC plate serving as the laminating card [1], while ensuring overlapping relation between each parts; the finished immunochromatographic chip can be used directly or loaded into a plastic cartridge for use; in a better manner, the PVC plate is cut into separate and usable finished products at the break points [17] between the test array units [12], so as to obtain the immunochromatographic chip in the invention.

12. A method for biological target detection with the multiassay immunochromatographic chip according to any one of claims 1-10, comprising:
A. Adding sample: adding a liquid sample or pretreated liquid sample in droplets onto the sample pad [2] of the immunochromatographic chip according to any one of claims 1-10;
B. Immunochromatographic reaction: standing for several minutes, till the immunochromatographic reaction is completed, wherein in the immunochromatographic reaction process, specific immunologic reactions occur among the test conjugates [6], the detected targets [10], and the solid-phase test probes [15], and the bonded amount of the marker [8] is changed at the well-defined fixed positions on the analysis membrane [4], and the bonded amount of the marker [8] at a certain position directly reflects the existence or concentration of a certain kind of detected target [10];
C. Result judgment: judging the result directly by visual examination for a color marker [8], or judging the result with instruments for a marker [8] that generates an optical, electrical, or magnetic signal, wherein, since one kind of solid-phase test probe [15] for a specific kind of detected target [10] is positioned fixedly at a well-defined position on the analysis membrane [4], this kind of detected target [10] can be detected qualitatively and quantitatively by judging the signals including a color, optical, electrical, or magnetic signal from the marker [8] fixed at the well-defined position.
